Europäisches Patentamt

European Patent Office

Office européen des brevets

(19)

(11) Veröffentlichungsnummer: **0 133 691**
**A2**

# (12) EUROPÄISCHE PATENTANMELDUNG

(21) Anmeldenummer: **84109210.9**

(22) Anmeldetag: **03.08.84**

(51) Int. Cl.⁴: **C 07 C 79/37**
**C 07 C 76/06**

(30) Priorität: **16.08.83 DE 3329453**

(43) Veröffentlichungstag der Anmeldung:
**06.03.85 Patentblatt 85/10**

(84) Benannte Vertragsstaaten:
**DE FR GB**

(71) Anmelder: **BAYER AG**
**Konzernverwaltung RP Patentabteilung**
**D-5090 Leverkusen 1 Bayerwerk(DE)**

(72) Erfinder: **Reinhardt, Karl-Heinz**
**Carl-von-Ossietzky-Strasse 10**
**D-5090 Leverkusen 1(DE)**

(72) Erfinder: **Stawitz, Josef, Dr.**
**Gerstenkamp 21**
**D-5000 Koeln 80(DE)**

(72) Erfinder: **Wunderlich, Klaus, Dr.**
**Carl-Rumpff-Strasse 21**
**D-5090 Leverkusen 1(DE)**

(54) **Verfahren zur Gewinnung von reinem 1-Nitroanthrachinon.**

(57) Verfahren zur Gewinnung von reinem 1-Nitroanthrachinon aus Roh-1-Nitroanthrachinon durch Behandeln des Roh-1-Nitroanthrachinons mit Nitrobenzol, gemäß dem man 1 Gewichtsteil eines mindestens 82 %-igen Roh-1-Nitroanthrachinons mit 0,5 - 5 Gew.-Teilen Nitrobenzol bei einer Temperatur von 100 - 200°C behandelt, die erhaltene Lösung oder Suspension auf 20 - 100°C abkühlt und aus dem ausgefallenen kristallisierten Produkt die in diesem enthaltenen Feinanteile mit Hilfe solcher an sich bekannten mechanischen Trennverfahren entfernt, die die unterschiedlichen Korngrößen und/oder Sinkgeschwindigkeiten der zu trennenden Feststoffe ausnutzen.

EP 0 133 691 A2

BAYER AKTIENGESELLSCHAFT    5090 Leverkusen, Bayerwerk
Konzernverwaltung RP
Patentabteilung           B/Kü-c

## Verfahren zur Gewinnung von reinem 1-Nitroanthrachinon

Die Erfindung betrifft ein neues Verfahren zur Gewinnung von reinem 1-Nitroanthrachinon durch Behandeln von Roh-1-Nitroanthrachinon in einem organischen Lösungsmittel und anschließender mechanischer Auftrennung des bei dieser Behandlung anfallenden, aus einem grobkristallinen und einem feinkristallinen Anteil bestehenden kristallisierten Produktes.

Bei der Mononitrierung von Anthrachinon entstehen neben dem erwünschten Hauptprodukt, 1-Nitroanthrachinon, größere Mengen an Nebenprodukten wie 2-Nitroanthrachinon und verschiedene Dinitroanthrachinone. Außerdem enthält das Nitroanthrachinon-Gemisch noch gewisse Mengen an nicht umgesetztem Anthrachinon. Ein solches stark verunreinigtes Nitroanthrachinon ist als Ausgangsprodukt für die Herstellung von Farbstoffen nicht verwendbar. Deshalb wurden zur Gewinnung von reinem 1-Nitroanthrachinon aus dem bei der Mononitrierung von Anthrachinon anfallenden Nitroanthrachinon-Gemisch bereits zahlreiche Reinigungsverfahren entwickelt. Diese lassen sich in folgende drei Gruppen unterteilen:

Le A 22 539

a. Reinigung des Nitroanthrachinon-Gemisches durch fraktionierte Kristallisation. Als Lösungsmittel wurden bei diesen Verfahren beispielsweise verwendet: Salpetersäure (DE-OS 2 253 016), Säureamide (DE-OS 2 039 822), Nitrobenzol (DE-OS 2 654 649), Mischungen von Salpetersäure und organischen Lösungsmitteln (DE-OS 2 400 253 und DE-OS 2 344 776).

b. Reinigung des Nitroanthrachinon-Gemisches durch chemische Behandlung. Ziel dieser Behandlung ist, die unerwünschten Nebenprodukte in leicht abtrennbare Verbindungen zu überführen, z.B. durch Behandlung mit Sulfiten (DE-OS 2 248 990, DE-OS 2 315 885, OS-PS 2 302 729), mit Reduktionsmitteln (DE-OS 2 415 662), mit basischen Verbindungen (DE-OS 2 304 233 und DE-OS 2 232 446), mit Phenolaten und Alkoholaten (DE-OS 2 233 076) oder Hydrazin (DE-OS 2 449 219).

c. Reinigung des Nitroanthrachinon-Gemisches oder eines Roh-1-Nitroanthrachinons (1-Nitroanthrachinon-Gehalt < 92 %) durch Destillation oder Rektifikation (DE-PS 281 490, DE-OS 2 256 644 und DE-OS 2 654 649).

Die durch Reinigungsverfahren der Gruppen a + b erzielten 1-Nitroanthrachinon-Qualitäten reichen zur Herstellung von Farbstoffen in den meisten Fällen nicht aus. Außerdem sind im allgemeinen die Ausbeuten an reinem 1-Nitroanthrachinon für eine wirtschaftliche Herstellung in technischem Maßstab nicht ausreichend.

Le A 22 539

Die Reinigungsverfahren der Gruppe b haben zusätzlich den Nachteil, daß die Nebenprodukte und in gewissem Umfang auch 1-Nitroanthrachinon irreversibel in andere Verbindungen überführt werden und damit ihre Verwertung, z.B. zur Herstellung von Dinitroanthrachinon, nicht mehr möglich ist.

Die Reinigung von rohem Nitroanthrachinon durch Destillation gemäß DE-PS 281 490 führt zu einem 1-Nitroanthrachinon, dessen Qualität für die Farbstoffherstellung nicht genügt. Dies ist durch die Anwesenheit von leichtflüchtigen Anthrachinonverbindungen, hauptsächlich Anthrachinon und 2-Nitroanthrachinon in dem zu destillierendem rohen 1-Nitroanthrachinon bedingt.

Die in den DE-OS 2 256 644 und DE-OS 2 654 649 beschriebenen Verfahren liefern zwar ein für die Farbstoffherstellung ausreichend reines 1-Nitroanthrachinon in guten Ausbeuten. Diese Verfahren haben jedoch den Nachteil, daß die Rektifikation des in bestimmter Weise gewonnenen Roh-1-Nitroanthrachinons wegen der erforderlichen hohen Temperaturen einen hohen apparativen und sicherheitstechnischen Aufwand erfordert.

Es wurde nun gefunden, daß man in einer sehr einfachen aber trotzdem äußerst wirkungsvollen Weise aus Roh-1-Nitroanthrachinon ein 1-Nitroanthrachinon gewinnen kann, dessen Reinheitsgrad den Anforderungen entspricht, die an ein für die Farbstoff-Herstellung verwendbares 1-Nitroanthrachinon gestellt werden,

Le A 22 539

wenn man ein Roh-1-Nitroanthrachinon, dessen Gehalt an bestimmten Nebenprodukten (Anthrachinon, 2-Nitroanthrachinon und Dinitroanthrachinonen) gewisse Werte nicht überschreitet, in an sich bekannter Weise mit einer gewissen Menge Nitrobenzol behandelt und aus dem nach dieser Behandlung anfallenden kristallisierten Produkt die in diesem enthaltenen Feinanteile mittels an sich bekannter mechanischer Trennmethoden entfernt.

Es wurde nämlich überraschenderweise gefunden, daß sich beim Behandeln von Roh-1-Nitroanthrachinon einer gewissen Zusammensetzung mit einer bestimmten Menge Nitrobenzol reines 1-Nitroanthrachinon und Nebenprodukt, vor allem 1,5-Dinitroanthrachinon, in getrennten Kristallen abscheiden, die sich in ihrer Größe und in ihrer Sinkgeschwindigkeit so stark von einander unterscheiden, daß man sie mit Hilfe an sich bekannter mechanischer Trennverfahren, die die unterschiedlichen Korngrößen und/ oder Sinkgeschwindigkeiten der zu trennenden Feststoffe ausnutzen, voneinander trennen kann.

Es wurde nämlich gefunden, daß 1-Nitroanthrachinon in groben Kristallen (mittlerer Teilchendurchmesser $>$ 400 µm) ausfällt, während der mittlere Teilchendurchmesser der in feinen Kriställchen ausfallenden Nebenprodukte $<$ 300 µm ist.

Die Erfindung betrifft daher ein Verfahren zur Gewinnung von reinem 1-Nitroanthrachinon aus Roh-1-Nitroanthrachinon durch Behandeln des Roh-1-Nitroanthrachinons mit Nitrobenzol, das dadurch gekennzeichnet ist, daß man 1 Gewichtsteil mindestens 82 %-iges Roh-1-Nitro-

Le A 22 539

anthrachinon, das weniger als 2 Gew.-% Anthrachinon, weniger als 2 Gew.-% 2-Nitroanthrachinon und weniger als 15 Gew.-% Dinitroanthrachinone enthält, mit 0,5 - 5 Gew.-Teilen Nitrobenzol, bei einer Temperatur von 100 - 200°C, vorzugsweise 120 - 180°C, behandelt, die erhaltene Lösung oder Suspension auf 20 - 100°C, vorzugsweise 65 - 95°C abkühlt, aus dem ausgefallenen kristallisierten Produkt, gegebenenfalls nach vorheriger Isolierung aus der Mutterlauge, die in ihm enthaltenen Feinanteile mittels solcher an sich bekannten mechanischen Trennverfahren entfernt, die die unterschiedlichen Korngrößen und/oder Sinkgeschwindigkeiten der zu trennenden Feststoffe ausnutzen, und das von den Feinanteilen d.h. den Nebenprodukten befreite, grob kristalline Produkt, d.h. das reine 1-Nitroanthrachinon, gegebenenfalls in bekannter Weise aus der Suspension isoliert und vom Lösungsmittel befreit.

Mit Hilfe des erfindungsgemäßen Verfahrens wird aus einem mindestens 82 %-igen 1-Nitroanthrachinon in guter Trennausbeute (85 - 90 %) und einem geringen Bedarf an Lösungsmittel ein 96 - 97,5 %-iges 1-Nitroanthrachinon erhalten, das den Anforderungen der Farbstoffherstellung überwiegend genügt. Unterwirft man dies bereits reine 1-Nitroanthrachinon nochmals dem erfindungsgemäßen Verfahren, so erhält man ein 98 - 99 %-iges 1-Nitroanthrachinon. Eine solche zweimalige Reinigung kann dann von Vorteil sein, wenn ein Roh-1-Nitroanthrachinon besonders schlechter Quali-

Le A 22 539

tät eingesetzt oder eine besonders hohe Reinheit verlangt wird. Abgesehen von seiner einfachen Ausführbarkeit hat das erfindungsgemäße Verfahren den Vorteil, daß die abgetrennten Nebenprodukte, insbesondere die Dinitroanthrachinone, nicht verloren gehen sondern in kristalliner Form anfallen und in dieser Form weiterverwertet werden können.

In dem erfindungsgemäßen Verfahren werden zur mechanischen Abtrennung der Feinanteile aus dem kristallisierten Produkt solche an sich bekannten mechanischen Trennverfahren angewendet, die die unterschiedliche Korngrößen und/oder unterschiedlichen Sinkgeschwindigkeiten der zu trennenden Feststoffe ausnutzen. Solche Verfahren sind z.B. die bekannten Klassierverfahren Sieben, Windsichten und Stromklassieren, ferner die Filtration unter Verwendung von Sieben einer Porengröße, die zwischen der Größe des grobkristallinen 1-Nitroanthrachinons und der der feinteiligen Nebenprodukte liegt. Auch spezielle Zentrifugen und Hydrozyklone können zur Abtrennung der feinteiligen Nebenprodukte vom grobkristallinen 1-Nitroanthrachinon verwendet werden. Die Verfahren können diskontinuierlich oder kontinuierlich durchgeführt werden. Sie sind z.B. in Ullmann's Enzyclopädie der technischen Chemie 4. Auflage, Band II auf Seiten 43, 57, 70, 154 und 204 beschrieben.

Das der mechanischen Abtrennung der Feinanteile vorangehende Behandeln des Roh-1-Nitroanthrachinons mit Nitrobenzol kann auf verschiedene Weise erfolgen, ent-

Le A 22 539

weder durch Suspendieren des Roh-1-Nitroanthrachinons im Nitrobenzol und Tempern der Suspension bei Temperaturen von 100 - 130°C (Arbeitsweise a), oder durch vollständiges Auflösen des Roh-1-Nitroanthrachinons im Nitrobenzol in der Wärme (vorzugsweise bei Temperaturen von 150 - 160°C Arbeitsweise (b)). Sowohl nach Arbeitsweise (a) als auch nach Arbeitsweise (b) wird ein qualitativ hochwertiges 1-Nitroanthrachinon in hoher Trennausbeute erhalten. Arbeitsweise (b) hat jedoch gegenüber Arbeitsweise (a) den Vorteil, daß die Qualität des erhaltenen 1-Nitroanthrachinons und die Trennausbeuten unabhängiger sind von der Qualität und der physikalischen Beschaffenheit des eingesetzten Roh-1-Nitroanthrachinons.

Das Nitrobenzol zum Suspendieren bzw. Auflösen des Roh-1-Nitroanthrachinons wird in einer Menge von 0,5 - 5 Gew.-Teilen je Gew.-Teil Roh-1-Nitroanthrachinon eingesetzt.

Anschließend an das Tempern bzw. Auflösen werden die Suspensionen bzw. Lösungen auf eine Temperatur von 20 - 100°C, vorzugsweise 60 - 95°C abgekühlt. Die Temperatur, auf die abgekühlt wird, richtet sich vor allem nach der verwendeten Menge an Nitrobenzol. Die Trennausbeute sinkt bei vorgegebener Temperatur bei Erhöhung der Lösungsmittelmenge oder bei vorgegebener Lösungsmittelmenge bei Temperaturerhöhung. Zur Erzielung möglichst hoher Trennausbeuten an reinem 1-Nitroanthrachinon werden unter Berücksichtigung der

Le A 22 539

Qualität des verwendeten Roh-1-Nitroanthrachinons Lösungsmittelmengen und Temperatur innerhalb der beanspruchten Bereiche so miteinander kombiniert, daß hohe Temperaturen bei niedrigen Lösungsmittelmengen, niedrige Temperaturen bei großen Lösungsmittelmengen und mittlere Temperaturen bei mittleren Lösungsmittelmengen angewendet werden.

Besonders bei Arbeitsweise (b) hat es sich als vorteilhaft erwiesen, wenn die heiße Lösung des Roh-1-Nitroanthrachinons in Nitrobenzol langsam und stufenweise, z.B. in Temperaturstufen von jeweils 10°C, auf die gewünschte Endtemperatur abgekühlt wird und auf jeder Temperaturstufe getempert wird. Auf diese Weise erhält man ein besonders gut klassierbares kristallisiertes Produkt, aus dem ein besonders reines 1-Nitroanthrachinon in besonders hoher Trennausbeute erhalten wird.

Das nach dem Abkühlen der Suspension bzw. Lösung angefallene kristallisierte Produkt wird entweder zunächst unaufgetrennt aus der Mutterlauge isoliert und dann mechanisch von den in ihm enthaltenen Feinanteilen befreit oder aber unmittelbar in der Mutterlauge der mechanischen Trennung unterworfen. Das kristallisierte Produkt wird unaufgetrennt aus der Mutterlauge nach an sich bekannten Verfahren, z.B. Filtrieren oder Schleudern, isoliert und anschließend getrocknet, wenn es von den Feinanteilen mittels eines mechanischen Trennverfahrens befreit werden soll, das ein getrocknetes Produkt erfordert, z.B. durch Sieben oder Windsichten. Soll dagegen das kristallisierte Produkt nach einem

Le A 22 539

mechanischen Trennverfahren von seinen Feinanteilen befreit werden, bei dem das aufzutrennende Feststoffgemisch in einer Flüssigkeit suspendiert vorliegen muß, z.B. durch Stromklassieren, Filtration oder Zentrifugieren, so kann man zwar auch in diesen Fällen das kristallisierte Produkt zunächst unaufgetrennt aus der Mutterlauge isolieren und erst nach erneutem Suspendieren in frischem Nitrobenzol besagter mechanischer Trennung unterwerfen; vorzugsweise wird jedoch das kristallisierte Produkt unmittelbar in der Mutterlauge durch Stromklassieren, Filtration oder Zentrifugieren von seinen Feinanteilen befreit. Besonders bewährt hat sich die unmittelbare Abtrennung der Feinanteile durch Aufstromklassierung. Bei dieser werden die Feinanteile mittels der Mutterlauge aus dem ausgefallenen kristallisierten Produkt ausgeschwemmt und als Trübe abgezogen. Die Trübe wird durch Filtration oder Zentrifugieren in den (im wesentlichen) aus den Nebenprodukten bestehenden feinkristallinen Niederschlag und Mutterlauge aufgetrennt. Nach dem Ausschwemmen der Feinanteile bleibt das grobkristalline, aus reinem 1-Nitroanthrachinon bestehende Produkt zurück.

Anstelle von Nitrobenzol kann man auch Mutterlauge (=Nitrobenzol-Lösung) verwenden, und zwar Mutterlauge, die im Verfahren nach dem Isolieren des kristallisierten Produktes bzw. nach dem Abtrennen der Feinanteile und dem Abfiltrieren bzw. Abschleudern des gereinigten 1-Nitroanthrachinons anfällt oder, falls das Reinigungsverfahren wiederholt wird,

Le A 22 539

Mutterlauge, die in dieser zweiten Reinigungsstufe anfällt. Durch diese Wiederverwendung der
Mutterlauge wird der Lösungsmittelverbrauch des erfindungsgemäßen Verfahrens wesentlich herabgesetzt.

Das nach dem mechanischen Abtrennen der Feinanteile
zurückbleibende grobkristalline 1-Nitroanthrachinon
wird, falls dies erforderlich ist, von der organischen
Lösung abgetrennt und vom anhaftenden Lösungsmittel
durch Absaugen und gegebenenfalls Waschen mit wenig
frischem Nitrobenzol befreit.

Sollte das auf diese Weise erhaltene reine 1-Nitroanthrachinon noch nicht den gewünschten Reinheitsgrad
aufweisen, so unterwirft man das bereits reine 1-Nitroanthrachinon noch einmal dem erfindungsgemäßen Reinigungsverfahren. Durch diese zweimalige Reinigung wird
auch aus einem nur 82 %-igen 1-Nitroanthrachinon ein
98 - 99 %-iges 1-Nitroanthrachinon erhalten.

Das in dem erfindungsgemäßen Verfahren als Ausgangsprodukt einzusetzende mindestens 82 %-ige Roh-1-Nitroanthrachinon, das weniger als 2 Gew.-% Anthrachinon,
weniger als 2 Gew.-% 2-Nitroanthrachinon und weniger
als 15 Gew.-% Dinitroanthrachinon enthält, wird durch
Umkristallisieren der Nitroanthrachinongemische aus
organischen Lösungsmitteln, vorzugsweise Nitrobenzol,
erhalten, die bei der Nitrierung von Anthrachinon mit
Salpetersäure gegebenenfalls in Gegenwart von Schwefelsäure anfallen.

Le A 22 539

Bevorzugt wird im erfindungsgemäßen Verfahren ein Roh-1-Nitroanthrachinon·eingesetzt, das man erhält, wenn man ein durch Nitrierung von Anthrachinon erhaltenes Nitroanthrachinon-Gemisch, das mindestens 60 Gew.-% 1-Nitroanthrachinon und weniger als 10 % Anthrachinon enthält, mit 0,8 - 3 Gew.-Teilen Nitrobenzol bei einer Temperatur von 120 - 180°C behandelt, die erhaltene Lösung oder Suspension auf etwa 100 - 20°C, vorzugsweise 100 - 70°C abkühlt; und das ausgefallene Roh-1-Nitroanthrachinon von der Mutterlauge abtrennt, z.B. abfiltriert oder abzentrifugiert.

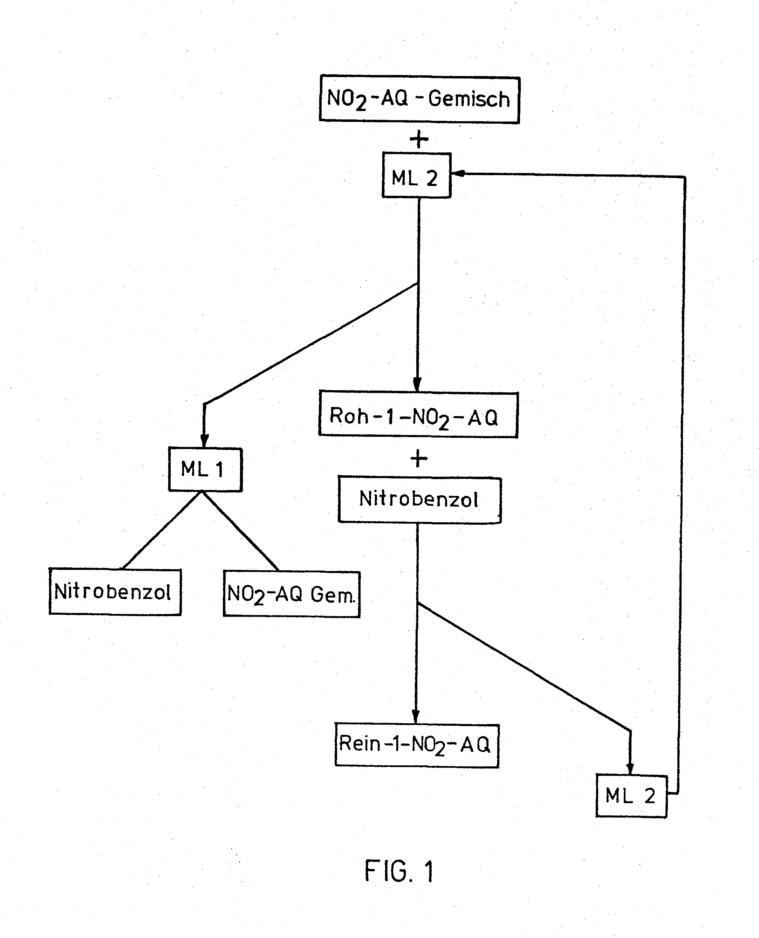

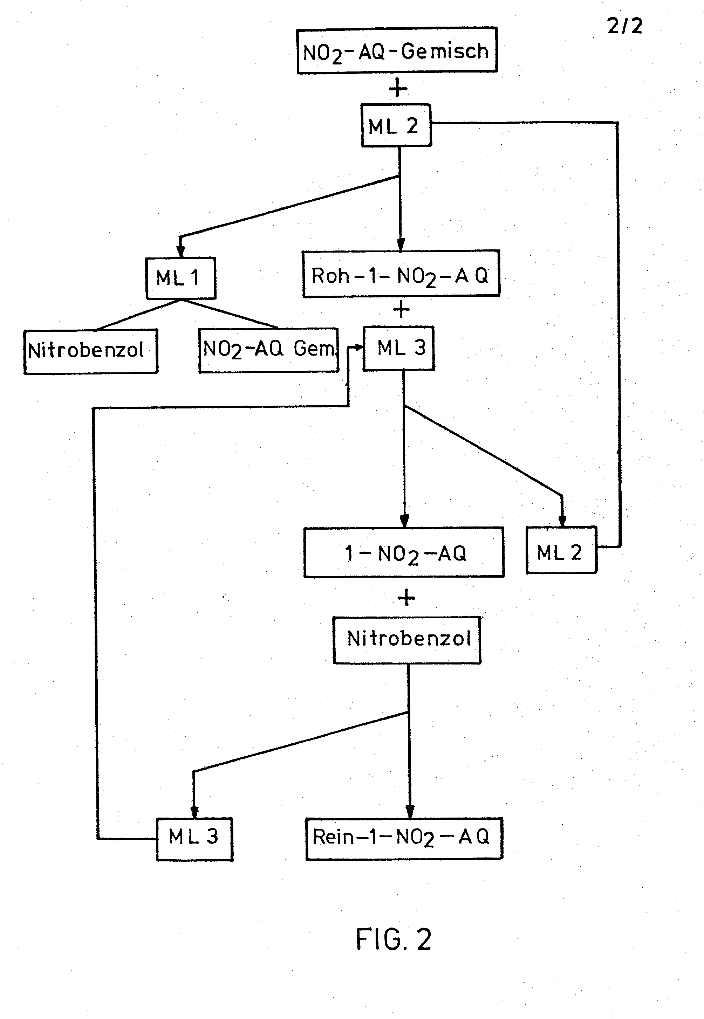

Da man in dieser Vorreinigungsstufe statt Nitrobenzol auch die in der nachfolgenden erfindungsgemäßen Reinigungsstufe anfallende Mutterlauge verwenden kann, gelingt es, unter sehr geringem Einsatz an Nitrobenzol aus einem 60 - 75 %-igen 1-Nitroanthrachinon ein 96 - 98,5 %-iges 1-Nitroanthrachinon in einer Trennausbeute von insgesamt 75 - 85 % zu erhalten. Bei der Verwendung von Mutterlauge zur Vorreinigung des Nitroanthrachinon-Gemisches wird die bei der Vorreinigung anfallende Mutterlauge anschließend aufgearbeitet. Die in diesem ausgeschleusten Teil enthaltenen Anthrachinonverbindungen werden durch Abdestillieren des Nitrobenzols isoliert und gegebenenfalls als Ausgangsstoffe für die Herstellung von Dinitroanthrachinon verwendet. Das Nitrobenzol kann ohne weitere Reinigung in irgendeine der Vorreinigungs- oder Reinigungsstufen eingesetzt werden. Im Hinblick auf die Bedeutung der Wiederverwendung der Mutterlaugen für die Wirtschaftlichkeit des erfindungsgemäßen Verfahrens sind zwei besonders vorteilhafte, in den Beispielen 4 und 5 erläuterte Möglichkeiten der Mutterlaugen-Wiederverwendung in Figur 1 und 2 schematisch dargestellt.

Le A 22 539

Beispiel 1

300 g Roh-1-Nitroanthrachinon folgender Zusammensetzung

| | |
|---|---|
| Anthrachinon | 0,32 Gew.-% |
| 2-Nitroanthrachinon | 0,94 Gew.-% |
| 2,6- u. 2,7-Dinitroanthrachinon | 0,27 Gew.-% |
| 1-Nitroanthrachinon | 85,77 Gew.-% |
| 1,6-Dinitroanthrachinon | 1,32 Gew.-% |
| 1,7-Dinitroanthrachinon | 3,25 Gew.-% |
| 1,5-Dinitroanthrachinon | 4,82 Gew.-% |
| 1,8-Dinitroanthrachinon | 1,74 Gew.-% |

werden in 470 g Nitrobenzol auf 160°C erhitzt bis eine klare Lösung entstanden ist. Diese läßt man unter Rühren innerhalb von 6 Stunden auf 70°C abkühlen, stellt dann den Rührer ab und läßt die groben Kristalle absetzen. Die überstehende trübe Mutterlauge gießt man über eine Nutsche ab und befreit sie damit von den Feinanteilen. Das klare, 70°C heiße Filtrat gibt man in den Kolben zurück, rührt kräftig durch, läßt die Grobanteile wieder absetzen und gießt die trübe Mutterlauge wieder über die gleiche Nutsche ab. Beim nochmaligen Anrühren der Grobanteile in der 70°C heißen klaren Mutterlauge bleibt diese praktisch klar. Nun werden die groben Kristalle abgesaugt und im Vakuum bei 120°C getrocknet.

Man erhält:

1. 236,3 g 1-Nitroanthrachinon (96,8 %ig) entsprechend einer Trennausbeute von 88 %

Le A 22 539

2.  13,6 g Feinanteile (Trockengewicht)

3.  477 g Mutterlauge mit einem Feststoffgehalt von etwa 10 Gew.-%.

Beispiel 2

Erste Reinigungsstufe:

300 g Roh-1-Nitroanthrachinon folgender Zusammensetzung

| | |
|---|---|
| Anthrachinon | 0,83 Gew.-% |
| 2-Nitroanthrachinon | 2,10 Gew.-% |
| 2,6- u. 2,7 -Dinitroanthrachinone | 0,27 Gew.-% |
| 1-Nitroanthrachinon | 83,45 Gew.-% |
| 1,6-Dinitroanthrachinon | 1,85 Gew.-% |
| 1,7-Dinitroanthrachinon | 4,38 Gew.-% |
| 1,5-Dinitroanthrachinon | 4,85 Gew.-% |
| 1,8-Dinitroanthrachinon | 2,20 Gew.-% |

werden in 470 g Mutterlauge aus Beispiel 1 auf 160°C erhitzt bis eine klare Lösung entstanden ist. Diese wird unter Rühren innerhalb von 5 Stunden auf 90°C abgekühlt, wobei man bei 140°C, 120°C, 100°C und 90°C die Suspension jeweils 1 Stunde tempert. Anschließend wird das ausgefallene kristalline Produkt wie in Beispiel 1 beschrieben bei 90°C von den Feinanteilen befreit. Das zurückbleibende grobkristalline Produkt wird abgesaugt und getrocknet.

Le A 22 539

Auf diese Weise werden erhalten:

1. 290 g Nitrobenzol-feuchtes 1-Nitroanthrachinon
   (etwa 94 %-iges 1-Nitroanthrachinon);

2. 446 g Mutterlauge mit einem Feststoffgehalt von
   etwa 16 Gew.-%;

3. 14,2 g Feinanteile (Trockengewicht)

Zweite Reinigungsstufe:

Die 290 g Nitrobenzol-feuchtes 1-Nitroanthrachinon
aus der ersten Reinigungsstufe werden in 330 g Nitrobenzol
auf 160°C erhitzt bis eine klare Lösung entstanden ist.
Diese wird innerhalb von 6 Stunden auf 65°C abgekühlt,
wobei man die Suspension bei 140°C, 120°C und 100°C
jeweils 1 Stunde tempert. Anschließend wird das ausgefallene kristalline Produkt wie in Beispiel 1 beschrieben von den Feinanteilen befreit.

Auf diese Weise werden erhalten:

1. 207,4 g 1-Nitroanthrachinon (98,5 %ig) entsprechend
   einer Trennausbeute von 82 %;

2. 16,1 g Feinanteile (Trockengewicht);

3. 350 g Mutterlauge mit einem Feststoffgehalt von
   etwa 10 Gew.-%.

Le A 22 539

Beispiel 3

a) Gewinnung des Roh-1-Nitroanthrachinons:

300 g Nitroanthrachinongemisch folgender Zusammensetzung

| | |
|---|---|
| Anthrachinon | 2,22 Gew.-% |
| 2-Nitroanthrachinon | 7,53 Gew.-% |
| 2,6- + 2,7-Dinitroanthrachinone | 0,76 Gew.-% |
| 1-Nitroanthrachinon | 74,13 Gew.-% |
| 1,6-Dinitroanthrachinon | 3,96 Gew.-% |
| 1,7-Dinitroanthrachinon | 3,84 Gew.-% |
| 1,5-Dinitroanthrachinon | 3,73 Gew.-% |
| 1,8-Dinitroanthrachinon | 3,38 Gew.-% |

werden in 360 g der in Beispiel 1 angefallenen Mutterlauge (Feststoffgehalt etwa 10 Gew.-%) bei 160°C gelöst. Die Lösung wird innerhalb von 3 Stunden auf 90°C abgekühlt und noch 3 Stunden auf dieser Temperatur gehalten. Anschließend wird der Niederschlag bei 90°C abgesaugt.

Auf diese Weise werden erhalten:

1.  246 g Nitrobenzol-feuchtes Roh-1-Nitroanthrachinon (Feststoffgehalt: 90 Gew.-%, etwa 90 %-iges 1-Nitroanthrachinon);

2.  398 g Mutterlauge (sie liefert nach Abdestillieren des Nitrobenzols 105,7 g Feststoff)

Le A 22 539

b) Reinigung des gewonnenen Roh-1-Nitroanthrachinons:

Die 246 g Nitrobenzol feuchtes Roh-1-Nitroanthrachinon aus der Vorreinigung (a) werden in 330 g Nitrobenzol auf 160°C erhitzt bis eine klare Lösung entstanden ist. Diese wird innerhalb von 7 Stunden auf 80°C abgekühlt, wobei man bei 140°C, 120°C, 100°C und 80°C die Suspension jeweils 1 Stunde tempert. Anschließend wird das ausgefallene kristalline Produkt wie in Beispiel 1 beschrieben von den Feinanteilen befreit. Das zurückbleibende grobkristalline Produkt wird abgesaugt und im Vakuum bei 120°C getrocknet:

Auf diese Weise werden erhalten:

1.  185,6 g 97,2 %-iges 1-Nitroanthrachinon entsprechend einer Trennausbeute von 81 % über beide Reinigungsstufen;

2.  6,9 g Feinanteile (Trockengewicht);

3.  345 g Mutterlauge mit einem Feststoffgehalt von etwa 9 Gew.-%.

Beispiel 4

a) Gewinnung des Roh-1-Nitroanthrachinons:

300 g Nitroanthrachinongemisch folgender Zusammensetzung

Le A 22 539

| Anthrachinon | 2,79 Gew.-% |
|---|---|
| 2-Nitroanthrachinon | 6,23 Gew.-% |
| 2,6- + 2,7-Dinitroanthrachinone | 0,71 Gew.-% |
| 1, -Nitroanthrachinon | 73,79 Gew.-% |
| 1,6-Dinitroanthrachinon | 4,16 Gew.-% |
| 1,7-Dinitroanthrachinon | 3,90 Gew.-% |
| 1,5-Dinitroanthrachinon | 4,09 Gew.-% |
| 1,8-Dinitroanthrachinon | 3,74 Gew.-% |

werden in etwa 360 g Mutterlauge aus der nachstehend unter b beschriebenen Reinigungsstufe auf 150 - 160°C erhitzt, bis eine klare Lösung entstanden ist. Diese wird unter Rühren innerhalb von 3 Stunden auf 90°C abgekühlt und drei Stunden auf dieser Temperatur nachgerührt. Anschließend wird der Niederschlag bei 90°C abgesaugt.

Auf diese Weise werden erhalten (Mittelwert aus 7 Reinigungscyclen):

1. 387 g Mutterlauge; sie liefert beim Destillieren 265 g reines Nitrobenzol und als Rückstand 100 g Nitroanthrachinongemisch, das für die Herstellung von Dinitroanthrachinon verwendet werden kann und

2. 272 g Nitrobenzol-feuchtes Roh-1-Nitroanthrachinon (Feststoffgehalt 83,1 Gew.-%) folgender Zusammensetzung:

Le A 22 539

| Anthrachinon | 0,36 Gew.-% |
| 2-Nitroanthrachinon | 1,18 Gew.-% |
| 2,6- + 2,7-Dinitroanthrachinone | |
| 1-Nitroanthrachinon | 87,97 Gew.-% |
| 1,6-Dinitroanthrachinon | 1,22 Gew.-% |
| 1,7-Dinitroanthrachinon | 1,70 Gew.-% |
| 1,5-Dinitroanthrachinon | 5,58 Gew.-% |
| 1,8-Dinitroanthrachinon | 1,99 Gew.-% |

b) Reinigung des gewonnenen Roh-1-Nitroanthrachinons:

Die 272 g Nitrobenzol-feuchtes Roh-1-Nitroanthrachinon werden in 320 g Nitrobenzol auf 160°C erhitzt bis eine klare Lösung entstanden ist. Die Lösung wird unter Rühren innerhalb von 6 Stunden auf 65°C abgekühlt, wobei man bei 140°C, 120°C und 100°C die Suspension jeweils 1 Stunde tempert. Anschließend wird das ausgefallene kristalline Produkt wie in Beispiel 1 beschrieben von den Feinanteilen befreit. Das zurückbleibende grobkristalline Produkt wird abgesaugt und getrocknet.

Auf diese Weise werden erhalten (Mittelwert aus 7 Reinigungscyclen):

1.    Etwa 360 g Mutterlauge, die vollständig als Lösungsmittel für das Nitroanthrachinongemisch in der Vor-reinigungsstufe a verwendet wird.

Le A 22 539

2.    16,3 g Feinanteile (Trockengewicht)

3.    175,5 g 96,9 %-iges 1-Nitroanthrachinon entsprechend einer Trennausbeute von 77 %.


Beispiel 5


a.    Gewinnung des Roh-1-Nitroanthrachinons


300 g des in Beispiel 4 beschriebenen durch Mononitrierung von Anthrachinon erhaltenen Nitroanthrachinongemisches werden in 370 g Mutterlauge aus der nachstehend unter (b) beschriebenen Reinigungsstufe auf 150 - 160°C erhitzt bis eine klare Lösung entstanden ist. Die Lösung wird innerhalb von 3 Stunden auf 90°C abgekühlt und 3 Stunden bei dieser Temperatur gerührt. Anschließend wird der Niederschlag bei 90°C abgesaugt.

Auf diese Weise werden erhalten (Mittelwert aus 9 Vorreinigungscyclen):

1.    339 g Mutterlauge; aus ihr werden durch Abdestillieren 240 g Nitrobenzol und 95 g Nitroanthrachinongemisch erhalten, das für die Herstellung von Dinitroanthrachinon verwendbar ist.

2.    325 g Nitrobenzol-feuchtes Roh-1-Nitroanthrachinon (Feststoffgehalt 79,6 %) folgender Zusammensetzung:


Le A 22 539

0133691

| Anthrachinon | 0,57 Gew.-% |
|---|---|
| 2-Nitroanthrachinon | 1,67 Gew.-% |
| 2,6- + 2,7-Dinitroanthrachinon | 0,27 Gew.-% |
| 1-Nitroanthrachinon | 82,87 Gew.-% |
| 1,6-Dinitroanthrachinon | 1,69 Gew.-% |
| 1,7-Dinitroanthrachinon | 5,49 Gew.-% |
| 1,5-Dinitroanthrachinon | 4,76 Gew.-% |
| 1,8-Dinitroanthrachinon | 2,14 Gew.-% |

b)    Reinigung des Roh-1-Nitroanthrachinons

Die 325 g Nitrobenzol-feuchtes Roh-1-Nitroanthrachinon aus Abschnitt (a) werden in 340 g Mutterlauge aus der nachstehend unter (c) beschriebenen zweiten Reinigungsstufe auf 160°C erhitzt, bis eine klare Lösung entstanden ist. Die Lösung wird innerhalb von 6 Stunden auf 90°C abgekühlt wobei man die Suspension jeweils 1 Stunde bei 140°C, 120°C, 100°C und 90°C tempert.

Das ausgefallene kristalline Produkt wird anschließend bei 90°C wie in Beispiel 1 beschrieben von den Feinanteilen befreit. Das so gereinigte grobkristalline Produkt wird abgesaugt.

Auf diese Weise werden erhalten (Durchschnittswert aus 9 Reinigungscyclen):

1.    370 g Mutterlauge (Feststoffgehalt etwa 15 Gew.-%),
       die vollständig in die unter Anschnitt a beschriebene
       Vorreinigung zurückgeführt wird.

Le A 22 539

2. 18,2 g Feinanteile (Trockengewicht)

3. 255 g Nitrobenzol-feuchtes Rein-1-Nitroanthrachinon (Feststoffgehalt etwa 80 %).

c) Reinigung des Rein-1-Nitroanthrachinons

Die 255 g des in der vorstehend beschriebenen Reinigungsstufe (b) erhaltenen feuchten reinen 1-Nitroanthrachinons werden in 300 g Nitrobenzol auf 160°C erhitzt bis eine klare Lösung entstanden ist. Die Lösung wird innerhalb von 6 Stunden auf 65°C abgekühlt wobei man die Suspension bei 140°C, 120°C und 100°C jeweils 1 Stunde tempert.

Das ausgefallene kristalline Produkt wird wie in Beispiel 1 beschrieben von den Feinanteilen befreit. Das zurückbleibende grobkristalline Produkt wird anschließend abgesaugt und getrocknet.

Auf diese Weise werden erhalten (Mittelwert aus 9 Reinigungscyclen):

1. 340 g Mutterlauge; sie wird als Lösungsmittel in der unter (b) beschriebenen Reinigungsstufe eingesetzt.

2. 8,7 g Feinanteile (Trockengewicht)

3. 168 g 1-Nitroanthrachinon (98 %-ig) entsprechend einer Trennausbeute von 74 %.

Le A 22 539

Beispiel 6

a)   Gewinnung des Roh-1-Nitroanthrachinons:

300 g Nitroanthrachinongemisch folgender Zusammensetzung

| | |
|---|---|
| Anthrachinon | 2,57 Gew.-% |
| 2-Nitroanthrachinon | 7,05 Gew.-% |
| 2,6- und 2,7-Dinitroanthrachinone | 0,75 Gew.-% |
| 1-Nitroanthrachinon | 72,93 Gew.-% |
| 1.6-Dinitroanthrachinon | 3,82 Gew.-% |
| 1.7-Dinitroanthrachinon | 3,85 Gew.-% |
| 1.5-Dinitroanthrachinon | 4,25 Gew.-% |
| 1.8-Dinitroanthrachinon | 4,08 Gew.-% |

werden in 168 g Nitrobenzol auf 170°C erhitzt, bis eine klare Lösung entstanden ist. Diese läßt man unter Rühren in 3 Stunden auf 95°C abkühlen und hält sie anschließend noch 3 Stunden bei dieser Temperatur. Anschließend wird der Niederschlag bei 95°C abgesaugt.

Auf diese Weise werden erhalten:

1.   267 g Nitrobenzol-feuchtes Roh-1-Nitroanthrachinon (Feststoffgehalt: 85 Gew.-%, etwa 88 %-iges 1-Nitroanthrachinon);

2.   200 g Mutterlauge (sie liefert nach Abdestillieren des Nitrobenzols 66,4 g Feststoff).

Le A 22 539

b)   Reinigung des gewonnenen Roh-1-Nitroanthrachinons:

Die 267 g Nitrobenzol-feuchtes Roh-1-Nitroanthrachinon aus der Vorreinigung (a) werden in 168 g Nitrobenzol auf 170°C erhitzt, bis eine klare Lösung entstanden ist. Diese wird innerhalb von 7 Stunden auf 95°C abgekühlt, wobei man die entstehende Suspension bei 150°C, 130°C, 110°C und 95°C jeweils 1 1/4 Stunden tempert. Anschließend wird das ausgefallene kristalline Produkt, wie in Beispiel 1 beschrieben, von den Feinanteilen befreit. Das zurückbleibende grobkristalline Produkt wird abgesaugt und im Vakuum bei 120°C getrocknet.

Auf diese Weise werden erhalten (Durchschnittswert aus 4 Versuchen):

1.   179,3 g 96,6 %-iges 1-Nitroanthrachinon entsprechend einer Trennausbeute von 79.2 % über beide Reinigungsstufen;
2.   8,0 g Feinanteile;
3.   215 g Mutterlauge (sie liefert nach Abdestillieren des Nitrobenzols 37,8 g Feststoff).

Le A 22 539

Beispiel 7

240 g 1-Nitroanthrachinon der folgenden Zusammensetzung

| | |
|---|---|
| Anthrachinon | 0,06 Gew.-% |
| 2-Nitroanthrachinon | 0,14 Gew.-% |
| 2.6- und 2.7-Dinitroanthrachinone | – |
| 1-Nitroanthrachinon | 96,92 Gew.-% |
| 1.6-Dinitroanthrachinon | 0,14 Gew.-% |
| 1.7-Dinitroanthrachinon | 0,10 Gew.-% |
| 1.5-Dinitroanthrachinon | 2,28 Gew.-% |
| 1.8-Dinitroanthrachinon | 0,36 Gew.-% |

werden in 240 g Nitrobenzol auf 170°C erhitzt, bis eine klare Lösung entstanden ist. Diese wird innerhalb von 7 Stunden auf 95°C abgekühlt, wobei man die entstehende Suspension bei 150°C, 130°C, 110°C und 95°C jeweils 1 1/4 Stunden tempert.

Das ausgefallene kristalline Produkt wird anschließend bei 95°C, wie in Beispiel 1 beschrieben, von den Feinteilen befreit.

Das so gereinigte grobkristalline Produkt wird mit der Mutterlauge noch einmal auf 170°C erhitzt, bis eine klare Lösung entstanden ist. Diese wird innerhalb von 7 Stunden auf 95°C abgekühlt, wobei man bei 150°C, 130°C, 110°C und 95°C die entstehende Suspension jeweils 1 1/4 Stunden tempert.

Das ausgefallene kristalline Produkt wird anschließend bei 95°C, wie in Beispiel 1 beschrieben, von den Feinteilen befreit. Das so gereinigte grobkristalline Produkt wird abgesaugt.

Le A 22 539

Auf diese Weise werden erhalten (Durchschnittswert aus 4 Versuchen):

1.  207,2 g 99,1 %-iges 1-Nitroanthrachinon entsprechend einer Trennausbeute von 88,3 % bezogen auf ca. 97 %-iges 1-Nitroanthrachinon;

2.  3,2 g Feinanteile (Trockengewicht von der 1. Klassierung);

3.  2,3 g Feinanteile (Trockengewicht von der 2. Klassierung);

4.  240 g Mutterlauge (sie liefert nach Abdestillieren des Nitrobenzols 25,4 g Feststoff.

Le A 22 539

Patentansprüche

1. Verfahren zur Gewinnung von reinem 1-Nitroanthrachinon aus Roh-1-Nitroanthrachinon durch Behandeln des Roh-1-Nitroanthrachinons mit Nitrobenzol, dadurch gekennzeichnet, daß man 1 Gewichtsteil eines mindestens 82 %-igen Roh-1-Nitroanthrachinons, das weniger als 2 Gew.-% Anthrachinon, weniger als 2 Gew.-% 2-Nitroanthrachinon und weniger als 15 Gew.-% Dinitroanthrachinone enthält, mit 0,5 - 5 Gew.-Teilen Nitrobenzol bei einer Temperatur von 100 - 200°C behandelt, die erhaltene Lösung oder Suspension auf 20 - 100°C abkühlt, aus dem ausgefallenen kristallisierten Produkt, gegebenenfalls nach vorheriger Isolierung aus der Mutterlauge, die in diesem enthaltenen Feinanteile mit Hilfe solcher an sich bekannten mechanischen Trennverfahren entfernt, die die unterschiedlichen Korngrößen und/oder Sinkgeschwindigkeiten der zu trennenden Feststoffe ausnutzen, und das von den Feinanteilen befreite grobkristalline Produkt (reines Nitroanthrachinon), gegebenenfalls in bekannter Weise isoliert und vom Lösungsmittel befreit.

2. Verfahren nach Anspruch 1 dadurch gekennzeichnet, daß man die Behandlung mit Nitrobenzol bei Temperaturen von 120 - 180°C vornimmt.

Le A 22 539

3. Verfahren nach Ansprüchen 1 oder 2 dadurch gekennzeichnet, daß man die erhaltene Lösung oder Suspension auf 60 - 95°C abkühlt.

4. Verfahren nach Ansprüchen 1, 2 oder 3 dadurch gekennzeichnet, daß man als mechanisches Trennverfahren
zur Abtrennung der Feinanteile die Stromklassierung
verwendet.

5. Verfahren nach Anspruch 4, dadurch gekennzeichnet,
daß man die Stromklassierung unmittelbar in der
nach der Lösungsmittelbehandlung anfallenden Suspension vornimmt.

6. Verfahren nach Ansprüchen 1 bis 5 dadurch gekennzeichnet, daß als Ausgangsprodukt ein Roh-1-Nitroanthrachinon verwendet wird, das man erhält, wenn
man ein mindestens 60 Gew.-% 1-Nitroanthrachinon
und weniger als 10 Gew.-% Anthrachinon enthaltendes
Nitroanthrachinon-Gemisch mit 0,8 - 3 Gew.-Teilen
Nitrobenzol bei einer Temperatur von 130 - 180°C
behandelt, die erhaltene Lösung oder Suspension
auf etwa 100 - 20°C abkühlt und das ausgefallene
Produkt von der Mutterlauge abtrennt.

7. Verfahren nach Ansprüchen 1 bis 6 dadurch gekennzeichnet, daß man zum Behandeln des Roh-1-Nitroanthrachinons anstelle von Nitrobenzol, die im
Verfahren anfallende Mutterlauge (Nitrobenzollösung) verwendet, oder, bei einer Wiederholung
des Verfahrens, die in dieser zweiten Reinigungsstufe anfallende Mutterlauge (Nitrobenzollösung).

Le A 22 539

FIG. 1

FIG. 2